# EUROPEAN PATENT APPLICATION

(11) **EP 4 296 360 A1**
(43) Date of publication of application: **27.12.2023**
(21) Application number: 22756311.1
(22) Date of filing: 18.02.2022
(51) Int. Cl.: C12N 15/54, C12N 1/15, C12N 1/19, C12N 1/21, C12N 5/10, C12N 9/10, C12P 19/18

(54) **MODIFIED PROTEIN HAVING ALPHA1,2-FUCOSYLTRANSFERASE ACTIVITY AND METHOD FOR PRODUCING FUCOSE-CONTAINING CARBOHYDRATE**

(30) Priority: 19.02.2021 JP 2021025170
(71) Applicant: Kyowa Hakko Bio Co., Ltd., Tokyo 100-8185 (JP)
(72) Inventor: TABATA, Kenichiro, Tokyo 100-8185 (JP); KAMADA, Nozomu, Tokyo 100-8185 (JP)
(74) Representative: Vossius & Partner Patentanwälte Rechtsanwälte mbB
(86) International application number: PCT/JP2022/006790
(87) International publication number: WO 2022/176994

(57) **Abstract**

An object of the present invention is to provide a modified protein having an α1,2-fucosyltransferase activity and a method for producing a fucose-containing carbohydrate using the protein or a microorganism having an ability to produce the protein. According to the present invention, a fucose-containing carbohydrate such as 2'-fucosyllactose can be more efficiently produced by using a protein having an α1,2-fucosyltransferase activity and being modified to substitute a specific amino acid residue with another amino acid residue, or a microorganism having the protein or an ability to produce the protein, compared to the case of using a wild-type protein having an α1,2-fucosyltransferase activity, or a microorganism having the protein or an ability to produce the protein.

## Description

### Technical Field

The present invention relates to a modified protein having an α1,2-fucosyltransferase activity and a method for producing a fucose-containing carbohydrate using the protein or a microorganism having an ability to produce the protein.

### Background Art

It has been reported that human milk oligosaccharides (HMO) contained in human milk have an effect of protecting infection from pathogenic bacteria and a function as a prebiotic, and from a physiological activity thereof, they are expected to be used as an additive for infant formula (Non Patent Literature 1).

Among carbohydrates known as HMOs, fucose-containing carbohydrates such as 2'-fucosyllactose account for about 60% of the total HMOs, and particular attention is paid to functionality of these carbohydrates (Non Patent Literature 2).

In a method for producing a fucose-containing carbohydrate by an enzyme reaction or fermentation production, α1,2-fucosyltransferase derived from Helicobacter pylori is widely used (Non Patent Literatures 2 and 3), but an activity of the fucosyltransferase is insufficient (Non Patent Literature 4).

In order to solve this problem, there has been known a method for producing a fucose-containing carbohydrate using α1,2-fucosyltransferase derived from Bacteroides fragilis (Non Patent Literature 5) or α1,2-fucosyltransferase derived from Helicobacter mustelae (Patent Literature 1), and development of a more efficient production method is required.

### Citation List

### Patent Literature

Patent Literature 1: Japanese Patent No. 4048173

### Non Patent Literature

Non Patent Literature 1: J Biotechnol (2016) 235: 61-83
Non Patent Literature 2: Curr Opin Biotechnol (2019) 56: 130-137
Non Patent Literature 3: Metabolic Engineering (2017) 41: 23-38
Non Patent Literature 4: Microb Cell Fact (2013) 12: 40
Non Patent Literature 5: Journal of Biotechnology (2017) 257: 192-198

### Summary of Invention

### Technical Problem

An object of the present invention is to provide a modified protein having an α1,2-fucosyltransferase activity and a method for producing a fucose-containing carbohydrate using the protein or a microorganism having an ability to produce the protein.

### Solution to Problem

The present invention relates to the following.
1. A protein having an improved α1,2-fucosyltransferase activity compared to an original protein according to any one of [1] to [3] below and consisting of an amino acid sequence in which one or more amino acid residues selected from the group consisting of amino acid residues corresponding to the amino acid residues at positions 9, 68, 75, 86, and 238 in the amino acid sequence represented by SEQ ID NO: 2 are substituted with another amino acid residue in an amino acid sequence of the protein according to any one of [1] to [3] below,
   [1] a protein consisting of the amino acid sequence represented by SEQ ID NO: 2, 4, 6, 8, or 10,
   [2] a mutant protein having an α1,2-fucosyltransferase activity and consisting of an amino acid sequence in which 1 to 20 amino acids are deleted, substituted, inserted, and/or added in the amino acid sequence represented by SEQ ID NO: 2, 4, 6, 8, or 10, and
   [3] a homologous protein having an α1,2-fucosyltransferase activity and consisting of an amino acid sequence having an identity of 80% or more with the amino acid sequence represented by SEQ ID NO: 2, 4, 6, 8, or 10.
2. The protein according to the above 1, in which the protein consists of an amino acid sequence in which the amino acid residues corresponding to the amino acid residues at positions 9, 68, 75, 86, and 238 in the amino acid sequence represented by SEQ ID NO: 2 are substituted with another amino acid residue in the amino acid sequence of the protein according to any one of [1] to [3].
3. The protein according to the above 1 or 2, in which the protein consists of an amino acid sequence with at least one substitution selected from the following [1a] to [1e] in the amino acid sequence of the protein according to any one of [1] to [3],
   [1a] the amino acid residue corresponding to the amino acid residue at position 9 in the amino acid sequence represented by SEQ ID NO: 2 being substituted with L-lysine or L-arginine,
   [1b] the amino acid residue corresponding to the amino acid residue at position 68 in the amino acid sequence represented by SEQ ID NO: 2 being substituted with L-lysine or L-arginine,
   [1c] the amino acid residue corresponding to the amino acid residue at position 75 in the amino acid sequence represented by SEQ ID NO: 2 being substituted with L-proline,
   [1d] the amino acid residue corresponding to the amino acid residue at position 86 in the amino acid sequence represented by SEQ ID NO: 2 being substituted with L-threonine or L-serine, and
   [1e] the amino acid residue corresponding to the amino acid residue at position 238 in the amino acid sequence represented by SEQ ID NO: 2 being substituted with L-leucine, L-isoleucine, or L-alanine.
4. The protein according to any one of the above 1 to 3, in which the protein consists of an amino acid sequence with at least one substitution selected from the following [2a] to [2e] in the amino acid sequence of the protein according to any one of [1] to [3],
   [2a] the amino acid residue corresponding to the amino acid residue at position 9 in the amino acid sequence represented by SEQ ID NO: 2 being substituted with L-arginine,
   [2b] the amino acid residue corresponding to the amino acid residue at position 68 in the amino acid sequence represented by SEQ ID NO: 2 being substituted with L-arginine,
   [2c] the amino acid residue corresponding to the amino acid residue at position 75 in the amino acid sequence represented by SEQ ID NO: 2 being substituted with L-proline,
   [2d] the amino acid residue corresponding to the amino acid residue at position 86 in the amino acid sequence represented by SEQ ID NO: 2 being substituted with L-serine, and
   [2e] the amino acid residue corresponding to the amino acid residue at position 238 in the amino acid sequence represented by SEQ ID NO: 2 being substituted with L-alanine.
5. The protein according to any one of the above 1 to 4, in which the protein consists of an amino acid sequence in which the amino acid residue corresponding to the amino acid residue at position 9 in the amino acid sequence represented by SEQ ID NO: 2 is substituted with L-arginine in the amino acid sequence of the protein according to any one of [1] to [3].
6. The protein according to any one of the above 1 to 4, in which the protein consists of an amino acid sequence in which the amino acid residue corresponding to the amino acid residue at position 238 in the amino acid sequence represented by SEQ ID NO: 2 is substituted with L-alanine in the amino acid sequence of the protein according to any one of [1] to [3].
7. The protein according to any one of the above 1 to 4, in which the protein consists of an amino acid sequence in which the amino acid residue corresponding to the amino acid residue at position 9 in the amino acid sequence represented by SEQ ID NO: 2 is substituted with L-arginine and the amino acid residue corresponding to the amino acid residue at position 238 in the amino acid sequence represented by SEQ ID NO: 2 is substituted with L-alanine in the amino acid sequence of the protein according to any one of [1] to [3].
8. A DNA encoding the protein according to any one of the above 1 to 7.
9. A recombinant DNA comprising the DNA according to the above 8.
10. A transformant obtained by transforming a host cell with the recombinant DNA according to the above 9.
11. A method for producing a fucose-containing carbohydrate, including: culturing a microorganism having an ability to produce the protein according to any one of the above 1 to 7 in a culture medium to produce a fucose-containing carbohydrate in a culture product.
12. A method for producing a fucose-containing carbohydrate, including: culturing a microorganism having an ability to produce the protein according to any one of the above 1 to 7 in a culture medium, using an obtained culture product or a processed product of the culture product as an enzyme source, and causing the enzyme source, GDP-fucose, and a receptor carbohydrate to exist in an aqueous medium to produce a fucose-containing carbohydrate in the aqueous medium.
13. The method for producing a fucose-containing carbohydrate according to the above 12, in which the receptor carbohydrate is lactose.
14. The production method according to any one of the above 11 to 13, in which the fucose-containing carbohydrate is 2'-fucosyllactose.

### Advantageous Effects of Invention

According to the present invention, a modified protein having an α1,2-fucosyltransferase activity and a method for producing a fucose-containing carbohydrate using the protein or a microorganism having an ability to produce the protein are provided.

### Description of Embodiments

### 1. Protein of Present Invention

A protein of the present invention is a protein according to any one of (1) to (7) below.
(1) A protein having an improved α1,2-fucosyltransferase activity compared to an original protein according to any one of [1] to [3] below and consisting of an amino acid sequence in which one or more amino acid residues selected from the group consisting of amino acid residues corresponding to the amino acid residues at positions 9, 68, 75, 86, and 238 in the amino acid sequence represented by SEQ ID NO: 2 are substituted with another amino acid residue in an amino acid sequence of the protein according to any one of [1] to [3],
   [1] a protein consisting of the amino acid sequence represented by SEQ ID NO: 2, 4, 6, 8, or 10,
   [2] a mutant protein having an α1,2-fucosyltransferase activity and consisting of an amino acid sequence in which 1 to 20 amino acids are deleted, substituted, inserted, and/or added in the amino acid sequence represented by SEQ ID NO: 2, 4, 6, 8, or 10, and
   [3] a homologous protein having an α1,2-fucosyltransferase activity and consisting of an amino acid sequence having an identity of 80% or more with the amino acid sequence represented by SEQ ID NO: 2, 4, 6, 8, or 10.
(2) The protein according to the above (1), in which the protein consists of an amino acid sequence in which the amino acid residues corresponding to the amino acid residues at positions 9, 68, 75, 86, and 238 in the amino acid sequence represented by SEQ ID NO: 2 are substituted with another amino acid residue in the amino acid sequence of the protein according to any one of [1] to [3].
(3) The protein according to the above (1) or (2), in which the protein consists of an amino acid sequence with at least one substitution selected from the following [1a] to [1d] in the amino acid sequence of the protein according to any one of [1] to [3],
   [1a] the amino acid residue corresponding to the amino acid residue at position 9 in the amino acid sequence represented by SEQ ID NO: 2 being substituted with L-lysine or L-arginine,
   [1b] the amino acid residue corresponding to the amino acid residue at position 68 in the amino acid sequence represented by SEQ ID NO: 2 being substituted with L-lysine or L-arginine,
   [1c] the amino acid residue corresponding to the amino acid residue at position 75 in the amino acid sequence represented by SEQ ID NO: 2 being substituted with L-proline,
   [1d] the amino acid residue corresponding to the amino acid residue at position 86 in the amino acid sequence represented by SEQ ID NO: 2 being substituted with L-threonine or L-serine, and
   [1e] the amino acid residue corresponding to the amino acid residue at position 238 in the amino acid sequence represented by SEQ ID NO: 2 being substituted with L-leucine, L-isoleucine, or L-alanine.
(4) The protein according to any one of the above (1) to (3), in which the protein consists of an amino acid sequence with at least one substitution selected from the following [2a] to [2e] in the amino acid sequence of the protein according to any one of [1] to [3],
   [2a] the amino acid residue corresponding to the amino acid residue at position 9 in the amino acid sequence represented by SEQ ID NO: 2 being substituted with L-arginine,
   [2b] the amino acid residue corresponding to the amino acid residue at position 68 in the amino acid sequence represented by SEQ ID NO: 2 being substituted with L-arginine,
   [2c] the amino acid residue corresponding to the amino acid residue at position 75 in the amino acid sequence represented by SEQ ID NO: 2 being substituted with L-proline,
   [2d] the amino acid residue corresponding to the amino acid residue at position 86 in the amino acid sequence represented by SEQ ID NO: 2 being substituted with L-serine, and
   [2e] the amino acid residue corresponding to the amino acid residue at position 238 in the amino acid sequence represented by SEQ ID NO: 2 being substituted with L-alanine.
(5) The protein according to any one of the above (1) to (4), in which the protein consists of an amino acid sequence in which the amino acid residue corresponding to the amino acid residue at position 9 in the amino acid sequence represented by SEQ ID NO: 2 is substituted with L-arginine in the amino acid sequence of the protein according to any one of [1] to [3].
(6) The protein according to any one of the above (1) to (4), in which the protein consists of an amino acid sequence in which the amino acid residue corresponding to the amino acid residue at position 238 in the amino acid sequence represented by SEQ ID NO: 2 is substituted with L-alanine in the amino acid sequence of the protein according to any one of [1] to [3].
(7) The protein according to any one of the above (1) to (4), in which the protein consists of an amino acid sequence in which the amino acid residue corresponding to the amino acid residue at position 9 in the amino acid sequence represented by SEQ ID NO: 2 is substituted with L-arginine and the amino acid residue corresponding to the amino acid residue at position 238 in the amino acid sequence represented by SEQ ID NO: 2 is substituted with L-alanine in the amino acid sequence of the protein according to any one of [1] to [3].

The amino acid residues corresponding to the amino acid residues at positions 9, 68, 75, 86, and 238 in the amino acid sequence represented by SEQ ID NO: 2 in the amino acid sequence of the original protein according to any one of [1] to [3] refers to respective amino acid residues that, when the amino acid sequence of the original protein is aligned with the amino acid sequence of SEQ ID NO: 2, are aligned at the same position as the amino acid residues at positions 9, 68, 75, 86, and 238 in the amino acid sequence represented by SEQ ID NO: 2.

Examples of the amino acid residues corresponding to the amino acid residues at positions 9, 68, 75, 86, and 238 in the amino acid sequence represented by SEQ ID NO: 2 in the amino acid sequence of the original protein of any one of [1] to [3] include the following amino acid residues.
(i) When the original protein is a protein consisting of the amino acid sequence represented by SEQ ID NO: 2, respective amino acid residues at positions 9, 68, 75, 86, and 238 in the amino acid sequence represented by SEQ ID NO: 2.
(ii) When the original protein is a protein consisting of the amino acid sequence represented by SEQ ID NO: 4, respective amino acid residues at positions 9, 68, 75, 84, and 243 in the amino acid sequence represented by SEQ ID NO: 4.
(iii) When the original protein is a protein consisting of the amino acid sequence represented by SEQ ID NO: 6, respective amino acid residues at positions 7, 66, 73, 88, and 247 in the amino acid sequence represented by SEQ ID NO: 6.
(iv) When the original protein is a protein consisting of the amino acid sequence represented by SEQ ID NO: 8, respective amino acid residues at positions 7, 53, 60, and 207 in the amino acid sequence represented by SEQ ID NO: 8. In the amino acid sequence represented by SEQ ID NO:8, there is no amino acid residue corresponding to the amino acid residue at position 86 in the amino acid sequence represented by SEQ ID NO:2.
(v) When the original protein is a protein consisting of the amino acid sequence represented by SEQ ID NO: 10, respective amino acid residues at positions 7, 66, 73, 82, and 238 in the amino acid sequence represented by SEQ ID NO: 10.

The fact that one or more amino acid residues selected from the group consisting of amino acid residues corresponding to the amino acid residues at positions 9, 68, 75, 86, and 238 in the amino acid sequence represented by SEQ ID NO: 2 are substituted with another amino acid residue in the amino acid sequence of the protein according to any one of [1] to [3] can be confirmed by, for example, aligning the amino acid sequence of the protein according to any one of [1] to [3] to be confirmed to be substituted with another amino acid residue with the amino acid sequence of the original protein.

The alignment of the amino acid sequences can be created by using, for example, a well-known alignment program ClustalW [Nucelic Acids Research 22, 4673, (1994)]. ClustalW is available, for example, from http://www. ebi.ac. uk/clustalw/ (European Bioinformatics Institute). For example, a default value can be used for parameters when creating an alignment using ClustalW.

The other amino acid residues described in the above (1) or (2) may be natural or non-natural. Examples of the natural amino acid include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, and L-cysteine.

Examples of mutually substitutable amino acids are shown below. Amino acids contained in the same group can be mutually substituted.
Group A: leucine, isoleucine, norleucine, valine, norvaline, alanine, 2-aminobutanoic acid, methionine, o-methylserine, t-butylglycine, t-butylalanine, and cyclohexylalanine
Group B: aspartic acid, glutamic acid, isoaspartic acid, isoglutamic acid, 2-aminoadipic acid, and 2-aminosuberic acid
Group C: asparagine and glutamine
Group D: lysine, arginine, omithine, 2,4-diaminobutanoic acid, and 2,3-diaminopropionic acid
Group E: proline, 3-hydroxyproline, and 4-hydroxyproline
Group F: serine, threonine, and homoserine
Group G: phenylalanine and tyrosine

As one embodiment of the protein described in the above (1) or (2), it is preferred that the substitution of an amino acid residue with another amino acid residue in the amino acid sequence of the protein according to any one of [1] to [3] includes at least one selected from the following [a] to [e], and it is more preferred that the other amino acid residue is an L-form.
[a] the amino acid residue corresponding to the amino acid residue at position 9 in the amino acid sequence represented by SEQ ID NO: 2 being substituted with one selected from amino acid residues described in the group D (lysine, arginine, ornithine, 2,4-diaminobutanoic acid, and 2,3-diaminopropionic acid)
[b] the amino acid residue corresponding to the amino acid residue at position 68 in the amino acid sequence represented by SEQ ID NO: 2 being substituted with one selected from amino acid residues described in the group D (lysine, arginine, ornithine, 2,4-diaminobutanoic acid, and 2,3-diaminopropionic acid)
[c] the amino acid residue corresponding to the amino acid residue at position 75 in the amino acid sequence represented by SEQ ID NO: 2 being substituted with one selected from amino acid residues described in the group E (proline, 3-hydroxyproline, and 4-hydroxyproline)
[d] the amino acid residue corresponding to the amino acid residue at position 86 in the amino acid sequence represented by SEQ ID NO: 2 being substituted with one selected from amino acid residues described in the group F (serine, threonine, and homoserine)
[e] the amino acid residue corresponding to the amino acid residue at position 238 in the amino acid sequence represented by SEQ ID NO: 2 being substituted with one selected from amino acid residues described in the group A (leucine, isoleucine, norleucine, valine, norvaline, alanine, 2-aminobutanoic acid, methionine, o-methylserine, t-butylglycine, t-butylalanine, and cyclohexylalanine)

As one embodiment of the protein described in the above (1) or (2), it is preferred that the substitution of an amino acid residue with another amino acid residue in the amino acid sequence of the protein according to any one of [1] to [3] includes at least one selected from the following [1a] to [1e].
[1a] the amino acid residue corresponding to the amino acid residue at position 9 in the amino acid sequence represented by SEQ ID NO: 2 being substituted with L-lysine or L-arginine,
[1b] the amino acid residue corresponding to the amino acid residue at position 68 in the amino acid sequence represented by SEQ ID NO: 2 being substituted with L-lysine or L-arginine,
[1c] the amino acid residue corresponding to the amino acid residue at position 75 in the amino acid sequence represented by SEQ ID NO: 2 being substituted with L-proline,
[1d] the amino acid residue corresponding to the amino acid residue at position 86 in the amino acid sequence represented by SEQ ID NO: 2 being substituted with L-threonine or L-serine, and
[1e] the amino acid residue corresponding to the amino acid residue at position 238 in the amino acid sequence represented by SEQ ID NO: 2 being substituted with L-leucine, L-isoleucine, or L-alanine.

As one embodiment of the protein described in the above (1) or (2), it is more preferred that the substitution of an amino acid residue with another amino acid residue in the amino acid sequence of the protein according to any one of [1] to [3] includes at least one selected from the following [2a] to [2e].
[2a] the amino acid residue corresponding to the amino acid residue at position 9 in the amino acid sequence represented by SEQ ID NO: 2 being substituted with L-arginine,
[2b] the amino acid residue corresponding to the amino acid residue at position 68 in the amino acid sequence represented by SEQ ID NO: 2 being substituted with L-arginine,
[2c] the amino acid residue corresponding to the amino acid residue at position 75 in the amino acid sequence represented by SEQ ID NO: 2 being substituted with L-proline,
[2d] the amino acid residue corresponding to the amino acid residue at position 86 in the amino acid sequence represented by SEQ ID NO: 2 being substituted with L-serine, and
[2e] the amino acid residue corresponding to the amino acid residue at position 238 in the amino acid sequence represented by SEQ ID NO: 2 being substituted with L-alanine.

As one embodiment of the protein described in the above (1) or (2), it is preferred that, among one or more amino acid residues selected from the group consisting of amino acid residues corresponding to the amino acid residues at positions 9, 68, 75, 86, and 238 in the amino acid sequence represented by SEQ ID NO: 2, at least one or more amino acid residue is substituted, and preferably two or more amino acid residues are substituted in the amino acid sequence of the protein according to [1] to [3].

As one embodiment of the protein described in the above (1) or (2), it is more preferred that the substitution of an amino acid residue with another amino acid residue in the amino acid sequence of the protein according to any one of [1] to [3] includes at least one of the following [A] and [B].
[A] the amino acid residue corresponding to the amino acid residue at position 9 in the amino acid sequence represented by SEQ ID NO: 2 being substituted with another amino acid residue, and preferably being substituted with one selected from amino acid residues described in the group D (lysine, arginine, omithine, 2,4-diaminobutanoic acid, and 2,3-diaminopropionic acid), and more preferably being substituted with L-arginine
[B] the amino acid residue corresponding to the amino acid residue at position 238 in the amino acid sequence represented by SEQ ID NO: 2 being substituted with another amino acid residue, preferably being substituted with one selected from amino acid residues described in the group A (leucine, isoleucine, norleucine, valine, norvaline, alanine, 2-aminobutanoic acid, methionine, o-methylserine, t-butylglycine, t-butylalanine, and cyclohexylalanine), and more preferably being substituted with L-alanine

As one embodiment of the protein described in the above (1), it is particularly preferred that the substitution of an amino acid residue with another amino acid residue in the amino acid sequence of the protein according to any one of [1] to [3] includes the following [C].
[C] the amino acid residue corresponding to the amino acid residue at position 9 in the amino acid sequence represented by SEQ ID NO: 2 being substituted with L-arginine and the amino acid residue corresponding to the amino acid residue at position 238 in the amino acid sequence represented by SEQ ID NO: 2 being substituted with L-alanine

The α1,2-fucosyltransferase activity refers to an activity of transferring fucose to a galactose residue of a receptor carbohydrate through α1,2 bonding using GDP-fucose and the receptor carbohydrate as substrates to produce a fucose-containing carbohydrate.

Examples of the receptor carbohydrate include a carbohydrate containing an oligosaccharide having galactose at a non-reducing end, preferably include a carbohydrate containing an oligosaccharide having a lactose, globotriose, N-acetyllactosamine, lact-N-tetraose, lact-N-neotetraose, Lewis X, or Lewis a structure at a non-reducing end, and more preferably include lactose, globotriose, N-acetyllactosamine, lacto-N-tetraose, lacto-N-neotetraose, Lewis X, or Lewis a.

Examples of the fucose-containing carbohydrate obtained using the receptor carbohydrate as a substrate include 2'-fucosyllactose, lacto-N-fucopentaose I, lactodifucotetraose, lacto-N-difucohexaose I, and lacto-N-neofucopentaose I.

It can be confirmed, for example, by the following method that the α1,2-fucosyltransferase activity of the protein according to any one of above (1) to (7) is improved as compared with the original protein. First, a recombinant DNA comprising a DNA encoding a protein whose activity is to be confirmed and a recombinant DNA comprising a DNA encoding an original protein are prepared, separately, by a method to be described later. Next, a microorganism having no α1,2-fucosyltransferase activity, for example, a microorganism obtained by transforming the Escherichia coli W3110 strain, is cultured with the recombinant DNA, and a cell extract containing the protein is prepared from the obtained culture product. The cell extract containing the protein is brought into contact with an aqueous solution containing GDP-fucose and a receptor carbohydrate as substrates to produce a fucose-containing carbohydrate in the aqueous solution. Finally, it can be confirmed that the α1,2-fucosyltransferase activity of the protein is improved as compared with the original protein by detecting the fucose-containing carbohydrate in the reaction liquid using a carbohydrate analyzer to be described later and comparing production amounts thereof.

The mutant protein refers to a protein obtained by artificially deleting or substituting an amino acid residue of an original protein or artificially inserting or adding an amino acid residue in the protein.

The expression "an amino acid is deleted, substituted, inserted, or added in the mutant protein" may mean that 1 to 20 amino acids are deleted, substituted, inserted, or added at any position in the same sequence.

An amino acid to be substituted, inserted, or added may be natural or non-natural. Examples of the natural amino acid include the above-described natural amino acids.

Examples of mutually substitutable amino acids are as described above. Amino acids contained in the same group can be mutually substituted.

The homologous protein refers to a group of proteins derived from proteins of the same evolutionary origin, which are proteins possessed by organisms existing in nature. The homologous proteins are similar in structure and function to each other.

The identity of amino acid sequences and nucleotide sequences can be determined using an algorithm BLAST [Pro. Nat. Acad. Sci. USA, 90, 5873, 1993] or FASTA [Methods Enzymol., 183, 63, 1990] according to Karlin and Altschul. Based on the algorithm BLAST, a program called BLASTN or BLASTX has been developed [J. Mol. Biol., 215, 403 (1990)]. When analyzing a nucleotide sequence using BLASTN based on BLAST, parameters are, for example, Score = 100 and wordlength = 12. When analyzing an amino acid sequence using BLASTX based on BLAST, parameters are, for example, score = 50 and wordlength = 3. When using BLAST and Gap ped BLAST programs, use default parameters for each program. A specific method of the analysis method is known.

It can be confirmed, for example, by the following method that the above mutant protein or homologous protein has an α1,2-fucosyltransferase activity. First, a recombinant DNA comprising a DNA encoding the above mutant protein or homologous protein whose activity is to be confirmed is prepared by a method to be described later. Next, a microorganism having no α1,2-fucosyltransferase activity, for example, a microorganism obtained by transforming the Escherichia coli W3110 strain, is cultured with the recombinant DNA, and a cell extract containing the protein is prepared from the obtained culture product. The cell extract containing the protein is brought into contact with an aqueous solution containing GDP-fucose and a receptor carbohydrate as substrates to produce a fucose-containing carbohydrate in the aqueous solution. Finally, it can be confirmed that the mutant protein or homologous protein has an α1,2-fucosyltransferase activity by detecting the fucose-containing carbohydrate in a reaction liquid using a carbohydrate analyzer to be described later.

Specific examples of the protein of the present invention as described above include proteins consisting of the amino acid sequence described in SEQ ID NO: 51,52, 53, or 54.

### 2. DNA of Present Invention

A DNA of the present invention is a DNA encoding the protein according to any one of above (1) to (7). Specific examples of the DNA of the present invention include any one of the following (8) to (13).

(8) A DNA encoding a protein consisting of an amino acid sequence in which one or more amino acid residues selected from the group consisting of amino acid residues corresponding to the amino acid residues at positions 9, 68, 75, 86, and 238 in the amino acid sequence represented by SEQ ID NO: 2 are substituted with another amino acid residue in an amino acid sequence of a protein encoded by a DNA according to any one of [4] to [6] below.
[4] A DNA consisting of the nucleotide sequence represented by SEQ ID NO: 1, 3, 5, 7, or 9
[5] A DNA hybridizing with a DNA consisting of a nucleotide sequence complementary to the nucleotide sequence represented by SEQ ID NO: 1, 3, 5, 7, or 9 under stringent conditions and encoding a homologous protein having an α1,2-fucosyltransferase activity
[6] A DNA consisting of a nucleotide sequence having an identity of at least 95% or more, preferably 97% or more, more preferably 98% or more, and most preferably 99% or more with the nucleotide sequence represented by SEQ ID NO: 1, 3, 5, 7, or 9 and encoding a homologous protein having an α1,2-fucosyltransferase activity

(9) A DNA according to the above (8), which encodes a protein consisting of an amino acid sequence in which an amino acid residue corresponding to the amino acid residue at position 9 in the amino acid sequence represented by SEQ ID NO: 2 is substituted with L-arginine, an amino acid residue corresponding to the amino acid residue at position 68 is substituted with L-arginine, an amino acid residue corresponding to the amino acid residue at position 75 is substituted with L-proline, an amino acid residue corresponding to the amino acid residue at position 86 is substituted with L-serine, or an amino acid residue corresponding to the amino acid residue at position 238 is substituted with L-alanine in the amino acid sequence encoded by the DNA.

(10) A DNA according to the above (8), which encodes a protein consisting of an amino acid sequence in which an amino acid residue corresponding to the amino acid residue at position 9 in the amino acid sequence represented by SEQ ID NO: 2 is substituted with L-arginine in the amino acid sequence encoded by the DNA.

(11) A DNA according to the above (8), which encodes a protein consisting of an amino acid sequence in which an amino acid residue corresponding to the amino acid residue at position 238 in the amino acid sequence represented by SEQ ID NO: 2 is substituted with L-alanine in the amino acid sequence encoded by the DNA.

(12) A DNA according to the above (8), which encodes a protein consisting of an amino acid sequence in which an amino acid residue corresponding to the amino acid residue at position 9 in the amino acid sequence represented by SEQ ID NO: 2 is substituted with L-arginine and an amino acid residue corresponding to the amino acid residue at position 238 is substituted with L-alanine in the amino acid sequence encoded by the DNA.

(13) A DNA comprising the nucleotide sequence represented by SEQ ID NO: 11, 12, 13, or 14.

In the above description, the term "hybridizing" refers to a step in which a DNA hybridizes to a DNA comprising a specific nucleotide sequence or a part of the DNA. Accordingly, a nucleotide sequence of the DNA hybridizing to a DNA comprising a specific nucleotide sequence or a part of the DNA may be useful as a probe for Northern or Southern blot analysis, or may be a DNA having a length that can be used as an oligonucleotide primer for PCR analysis.

Examples of the DNA used as a probe include DNAs having at least 100 bases or more, preferably 200 bases or more, and more preferably 500 bases or more. Examples of the DNA used as a primer include DNAs having at least 10 bases or more, and preferably 15 bases or more.

A method for a DNA hybridization experiment is well known, and for example, Molecular Cloning, 4th Edition (Cold Spring Harbor Laboratory Press (2012)), Methods for General and Molecular Bacteriology (ASM Press (1994)), Immunology methods manual (Academic press (1997)), and many other standard textbooks can be followed to determine hybridization conditions and perform experiments.

The DNA hybridizing under stringent conditions can also be obtained by following an explanatory manual attached to a commercially available hybridization kit. Examples of the commercially available hybridization kit include a random primed DNA labeling kit (manufactured by Roche Diagnostics K.K.) in which a probe is prepared by a random prime method and hybridization is performed under stringent conditions.

Examples of the above stringent conditions include conditions where a filter on which a DNA is immobilized and a probe DNA are incubated overnight at 42°C in a solution containing 50% formamide, 5×SSC (750 mM sodium chloride, 75 mM sodium citrate), 50 mM sodium phosphate (pH 7.6), a 5× Denhardt's solution, 10% dextran sulfate, and a 20 µg/L of denatured salmon sperm DNA, and then the filter is washed in a 0.2×SSC solution at, for example, about 65°C.

The above various conditions can also be set by adding or changing a blocking reagent used to reduce background in a hybridization experiment. The addition of the above blocking reagent may be accompanied by a change in hybridization conditions in order to meet the conditions.

Examples of the DNA capable of hybridizing under the above stringent conditions include a DNA consisting of a nucleotide sequence having an identity of at least 95% or more, preferably 97% or more, more preferably 98% or more, and most preferably 99% or more with the nucleotide sequence represented by SEQ ID NO: 1, 3, 5, 7, or 9 when calculated based on the above parameters using the above program such as BLAST or FAST A.

The DNA of the present invention can be obtained, for example, by using a DNA encoding a protein consisting of the amino acid sequence represented by SEQ ID NO: 2, 4, 6, 8, or 10, introducing a mutation to a nucleotide sequence of the moiety encoding one or more amino acid residues selected from the group consisting of amino acid residues corresponding to the amino acid residues at positions 9, 68, 75, 86, and 238 in the SEQ ID NO: 2 located on the DNA by a site-directed mutagenesis method described in, for example, Molecular Cloning 4th Edition (Cold Spring Harbor Laboratory Press, (2012)) and Current Protocols in Molecular Biology (JOHN WILEY & SONS, INC.), and substituting with a nucleotide sequence encoding another amino acid residue. Alternatively, the DNA of the present invention can also be obtained by using a PrimeSTAR Mutagenesis Basal Kit (manufactured by Takara Bio Inc.), or the like.

By the same method, the DNA of the present invention can also be obtained, for example, by using a DNA encoding a mutant protein having an α1,2-fucosyltransferase activity and comprising an amino acid sequence in which 1 to 20 amino acids are deleted, substituted, inserted, and/or added in the amino acid sequence represented by SEQ ID NO: 2, 4, 6, 8, or 10, and introducing a mutation to a nucleotide sequence of the moiety encoding one or more amino acid residues selected from the group consisting of amino acid residues corresponding to the amino acid residues at positions 9, 68, 75, 86, and 238 in the SEQ ID NO: 2 in the amino acid sequence of the mutant protein when the amino acid sequence of the mutant protein and the original amino acid sequence represented by SEQ ID NO: 2, 4, 6, 8 or 10 are aligned by the method described in the above 1.

The DNA of the present invention can also be obtained, for example, by using a DNA encoding a homologous protein having an α1,2-fucosyltransferase activity and consisting of an amino acid sequence having an identity of 90% or more with the amino acid sequence represented by SEQ ID NO: 2, 4, 6, 8, or 10, and introducing a mutation to a nucleotide sequence of the moiety encoding one or more amino acid residues among amino acid residues selected from the group consisting of amino acid residues corresponding to the amino acid residues at positions 9, 68, 75, 86, and 238 in the SEQ ID NO: 2 in the amino acid sequence of the homologous protein when the amino acid sequence of the homologous protein and the original amino acid sequence represented by SEQ ID NO: 2, 4, 6, 8 or 10 are aligned by the method described in the above 1.

The DNA encoding the protein consisting of the amino acid sequence represented by SEQ ID NO: 2 can be obtained, for example, by Southern hybridization to a chromosomal DNA library of a microorganism, preferably of the genus Helicobacter microorganism, and more preferably of the Helicobacter mustelae ATCC43772 strain using a probe that can be designed based on the nucleotide sequence of the DNA encoding the protein consisting of the amino acid sequence represented by SEQ ID NO: 2, or by PCR [PCR Protocols, Academic Press (1990)] using a primer DNA that can be designed based on the DNA encoding the protein consisting of the amino acid sequence represented by SEQ ID NO: 2 and using, as a template, a chromosomal DNA of the Helicobacter mustelae ATCC43772 strain. Specific examples of the DNA encoding the protein consisting of the amino acid sequence represented by SEQ ID NO: 2 include a DNA comprising the nucleotide sequence represented by SEQ ID NO: 1.

The DNA encoding the amino acid sequence represented by SEQ ID NO: 4 can be obtained, for example, by Southern hybridization to a chromosomal DNA library of a microorganism, preferably of the genus Helicobacter microorganism, and more preferably of the Helicobacter pylori ATCC700392 strain using a probe that can be designed based on the nucleotide sequence of the DNA encoding the protein consisting of the amino acid sequence represented by SEQ ID NO: 4, or by PCR using a primer DNA that can be designed based on the DNA encoding the protein consisting of the amino acid sequence represented by SEQ ID NO: 4 and using, as a template, a chromosomal DNA of the Helicobacter pylori ATCC700392 strain. Specific examples of the DNA encoding the protein consisting of the amino acid sequence represented by SEQ ID NO: 4 include a DNA comprising the nucleotide sequence represented by SEQ ID NO: 3.

The DNA encoding the amino acid sequence represented by SEQ ID NO: 6 can be obtained, for example, by Southern hybridization to a chromosomal DNA library of a microorganism, preferably of the genus Escherichia microorganism, and more preferably of the Escherichia coli O126 strain using a probe that can be designed based on the nucleotide sequence of the DNA encoding the protein consisting of the amino acid sequence represented by SEQ ID NO: 6, or by PCR using a primer DNA that can be designed based on the DNA encoding the protein consisting of the amino acid sequence represented by SEQ ID NO: 6 and using, as a template, a chromosomal DNA of the Escherichia coli O126 strain. Specific examples of the DNA encoding the protein consisting of the amino acid sequence represented by SEQ ID NO: 6 include a DNA comprising the nucleotide sequence represented by SEQ ID NO: 5.

The DNA encoding the amino acid sequence represented by SEQ ID NO: 8 can be obtained, for example, by Southern hybridization to a chromosomal DNA library of a microorganism, preferably of the genus Bacteroides microorganism, and more preferably of the Bacteroides fragilis ATCC25285 strain using a probe that can be designed based on the nucleotide sequence of the DNA encoding the protein consisting of the amino acid sequence represented by SEQ ID NO: 8, or by PCR using a primer DNA that can be designed based on the DNA encoding the protein consisting of the amino acid sequence represented by SEQ ID NO: 8 and using, as a template, a chromosomal DNA of the Bacteroides fragilis ATCC25285 strain. Specific examples of the DNA encoding the protein consisting of the amino acid sequence represented by SEQ ID NO: 8 include a DNA comprising the nucleotide sequence represented by SEQ ID NO: 7.

The DNA encoding the amino acid sequence represented by SEQ ID NO: 10 can be obtained by, for example, Southern hybridization to a chromosomal DNA library of a microorganism, preferably of the genus vibrio microorganism, and more preferably of the Vibrio cholerae O22 strain using a probe that can be designed based on the nucleotide sequence of the DNA encoding the protein consisting of the amino acid sequence represented by SEQ ID NO: 10, or by PCR using a primer DNA that can be designed based on the DNA encoding the protein consisting of the amino acid sequence represented by SEQ ID NO: 10 and using, as a template, a chromosomal DNA of the Vibrio cholerae O22 strain. Specific examples of the DNA encoding the protein consisting of the amino acid sequence represented by SEQ ID NO: 10 include a DNA comprising the nucleotide sequence represented by SEQ ID NO: 9.

The DNA encoding a mutant protein having an α1,2-fucosyltransferase activity and consisting of an amino acid sequence in which 1 to 20 amino acids are deleted, substituted, inserted, and/or added in the amino acid sequence represented by SEQ ID NO: 2, 4, 6, 8, or 10 according to the above 1(1)[2] can be obtained, for example, by subjecting a DNA consisting of the nucleotide sequence represented by SEQ ID NO: 1, 3, 5, 7 or 9 to error-prone PCR or the like as a template.

Alternatively, the DNA encoding the mutant protein having an α1,2-fucosyltransferase activity and consisting of an amino acid sequence in which 1 to 20 amino acids are deleted, substituted, inserted, or added in the amino acid sequence represented by SEQ ID NO: 2, 4, 6, 8, or 10 according to the above 1(1)[2] can also be obtained by a partially directed mutagenesis method by PCR using a set of PCR primers each having a nucleotide sequence designed to introduce a desired mutation (deletion, substitution, insertion, or addition) at the 5' end [Gene, 77, 51 (1989)].

The DNA can also be obtained by following an explanatory manual attached to a commercially available partially directed mutagenesis kit. Examples of the commercially available partially directed mutagenesis kit include a PrimeSTAR (registered trademark) Mutagenesis Basal Kit (manufactured by Takara Bio Inc.) capable of introducing a mutation (deletion, substitution, insertion, or addition) at a position to which a desired mutation is to be introduced.

That is, first, a pair of mutagenesis primers having a 15-base overlap on the 5' side is designed using a plasmid comprising a nucleotide sequence designed to introduce a desired mutation (deletion, substitution, insertion, or addition) as a template. At this time, the overlap portion includes a desired mutation. Next, PCR is performed using the mutagenesis primers and using a plasmid comprising a nucleotide sequence into which a desired mutation is introduced as a template. When the amplified fragment thus obtained is transformed into Escherichia coli, a plasmid comprising a nucleotide sequence into which a desired mutation is introduced can be obtained.

The DNA encoding the homologous protein having an α1,2-fucosyltransferase activity and consisting of an amino acid sequence having an identity of 80% or more with the amino acid sequence represented by SEQ ID NO: 2, 4, 6, 8, or 10 according to the above 1(1)[3] can be obtained, for example, by the following method. Specifically, for example, the DNA encoding the homologous protein can be obtained by a method similar to the method for obtaining the DNA encoding the protein consisting of the amino acid sequence represented by SEQ ID NO:2, 4, 6, 8, or 10, using a probe DNA or primer DNA that can be designed based on a nucleotide sequence or an amino acid sequence obtained by searching various gene sequence databases for a nucleotide sequence having an identity of 80% or more, preferably 90% or more, more preferably 95% or more, and most preferably 99% or more with the nucleotide sequence represented by SEQ ID NO: 1, 3, 5, 7, or 9 and a microorganism having the DNA.

The identity of the nucleotide sequence and the amino acid sequence can be determined by a method same as that described in the above 1.

A nucleotide sequence of the DNA of the present invention obtained by the above method can be determined by using the DNA as it is or cleaving the DNA with an appropriate restriction enzyme or the like, incorporating the DNA into a vector by an ordinary method, introducing the obtained recombinant DNA into a host cell, and then analyzing the DNA using a nucleotide sequence analysis method generally used, such as a dideoxy method [Proc. Nat. Acad. Sci., USA, 74, 5463 (1977)] or a nucleotide sequence analyzer such as an Applied Biosystems 3500 Genetic Analyzer and an Applied Biosystems 3730 DNA Analyzer (both manufactured by Thermo Fisher Scientific K.K.).

Examples of the vector that can be used for determining the nucleotide sequence of the DNA of the present invention include pBluescriptII KS(+) and pPCR-Script Amp SK(+) (both manufactured by Agilent Technologies, Inc.), pT7Blue (manufactured by Merck Millipore Inc.), pCRII (manufactured by Thermo Fisher Scientific K.K.), pCR-TRAP (manufactured by Gene Hunter), and pDIRECT [Nucleic Acids Res., 18, 6069 (1990)].

The above host cell may be any cell as long as the vector can be introduced and proliferated, and examples thereof include Escherichia coli DH5α, Escherichia coli HST08 Premium, Escherichia coli HST02, Escherichia coli HST04 dam⁻/dcm⁻, Escherichia coli JM109, Escherichia coli HB101, Escherichia coli CJ236, Escherichia coli BMH71-18 mutS, Escherichia coli MV1184, and Escherichia coli TH2 (all manufactured by Takara Bio Inc.), Escherichia coli XL1-Blue and Escherichia coli XL2-Blue (both manufactured by Agilent Technologies, Inc.), Escherichia coli DH1, Escherichia coli MC1000, Escherichia coli W1485, Escherichia coli W3110, Escherichia coli MP347, and Escherichia coli NM522.

As a method for introducing the recombinant DNA obtained by incorporating the DNA of the present invention into a host cell, any method for introducing a DNA into a host cell can be used, and examples thereof include a method using calcium ions [Proc. Natl. Acad. Sci., USA, 69, 2110 (1972)], a protoplast method (JPS63-248394A), and an electroporation method [Nucleic Acids Res., 16, 6127 (1988)].

When the obtained DNA is partial length as a result of determining the nucleotide sequence, the full-length DNA can be obtained by a Southern hybridization method or the like for a chromosomal DNA library using the partial length DNA as a probe.

Further, a target DNA can also be prepared by chemical synthesis using an NTS M series DNA synthesizer or the like manufactured by Nihon Techno Service Co., Ltd. based on the determined DNA nucleotide sequence.

### 3. Recombinant DNA of Present Invention

A recombinant DNA of the present invention is a DNA autonomously replicatable in a host cell, and is obtained by incorporating the DNA of the present invention of the above 2 into an expression vector comprising a promoter at a position where the DNA of the present invention can be transferred.

A DNA capable of being incorporated into a chromosome in a host cell and comprising the DNA of the present invention is also the recombinant DNA of the present invention.

When the recombinant DNA is a recombinant DNA capable of being incorporated into a chromosome, the recombinant DNA may not contain a promoter.

When a prokaryote such as bacteria is used as a host cell, the recombinant DNA of the present invention is preferably a recombinant DNA composed of a promoter, a ribosomal binding sequence, the DNA of the present invention of the above 2, and a transcription termination sequence. Further, a gene controlling a promoter may be comprised.

Here, it is preferred to adjust a distance between a shine-dalgarno sequence, which is a ribosomal binding sequence, and an initiation codon to an appropriate distance, for example, 6 to 18 bases.

In the recombinant DNA of the present invention, the transcription termination sequence is not necessarily required for the expression of the DNA of the present invention, but it is preferred to place a transcription termination sequence immediately below the structural gene.

When a microorganism belonging to the genus Escherichia is used as a host cell into which the recombinant DNA of the present invention is to be introduced, examples of the expression vector include pColdI, pSTV28, and pUC118 (all manufactured by Takara Bio Inc.), pET21a, pCDF-1b, and pRSF-1b (all manufactured by Merck Millipore Inc.), pMAL-c5x (manufactured by New England Biolabs), pGEX-4T-1 and pTrc99A (both manufactured by GE Healthcare Bioscience), pTrcHis and pSE280 (both manufactured by Thermo Fisher Scientific K.K.), pGEMEX-1 (manufactured by Promega Corporation), pQE-30, pQE-60, and pQE80L (all manufactured by Qiagen), pET-3, pBluescriptII SK(+), and pBluescriptII KS(-) (all manufactured by Agilent Technologies, Inc.), pKYP10 (JPS58-110600A), pKYP200 [Agric. Biol. Chem., 48, 669 (1984)], pLSA1 [Agric. Biol. Chem., 53, 277 (1989)], pGEL1 [Proc. Natl. Acad. Sci., USA, 82, 4306 (1985)], pTrS30 [prepared from Escherichia coli JM109/pTrS30 (FERM BP-5407)], pTrS32 [prepared from Escherichia coli JM109/pTrS32 (FERM BP-5408)], pTK31 [APPLIED AND ENVIRONMENTAL MICROBIOLOGY, 2007, Vol. 73, No. 20, p6378-6385], pPE167 (Appl. Environ. Microbiol. 2007, 73: 6378-6385), pPAC31 (WO 1998/12343), pUC19 [Gene, 33, 103 (1985)], and pPA1 (JPS63-233798A).

The promoter in the case of using the above expression vector may be any promoter as long as it functions in cells of a microorganism belonging to the genus Escherichia, and a promoter derived from Escherichia coli, phage, or the like, such as a trp promoter, a gapA promoter, a lac promoter, a PL promoter, a PR promoter, or a PSE promoter can be used. A promoter which is artificially modified in design, such as a promoter with two trp promoters in series, a tac promoter, a trc promoter, a lacT5 promoter, a lacT7 promoter, or a let I promoter can be used.

When a coryneform bacterium is used as a host cell into which the recombinant DNA of the present invention is to be introduced, examples of the expression vector include pCG1 (JPS57-134500A), pCG2 (JPS58-35197A), pCG4 (JPS57-183799A), pCG11 (JPS57-134500A), pCG 116, pCE54, and pCB101 (all JPS58-105999A), pCE51, pCE52, and pCE53 [all Molecular and General Genetics, 196, 175 (1984)].

When the above expression vector is used, the promoter may be any promoter as long as it functions in cells of the coryneform bacterium, and for example, a P54-6 promoter [Appl. Microbiol. Biotechnol., 53, p674-679 (2000)] can be used.

When a yeast strain is used as a host cell into which the recombinant DNA of the present invention is to be introduced, examples of the expression vector include YEp13 (ATCC37115), YEp24 (ATCC37051), YCp50 (ATCC37419), pHS19, and pHS15.

The promoter in the case of using the above expression vector may be any promoter as long as it functions in cells of the yeast strain, and examples thereof include a PHOS promoter, a PGK promoter, a GAP promoter, an ADH promoter, a gall promoter, a gal10 promoter, a heat shock polypeptide promoter, an MFα1 promoter, and a CUP1 promoter.

The recombinant DNA of the present invention can be prepared by, for example, subjecting the DNA fragment prepared by the method of the above 2 to a restriction enzyme treatment or the like and inserting the DNA fragment into downstream of the promoter of the appropriate expression vector.

Here, by substituting a base such that the nucleotide sequence of the DNA of the present invention is an optimal codon for expression in a host cell, the expression level of the protein encoded by the DNA can be improved. Information on the frequency of codon use in a host cell can be obtained through a public database.

### 4. Transformant of Present Invention

A transformant of the present invention is a transformant obtained by transforming a host cell with the recombinant DNA comprising the DNA of the present invention according to the above 2.

The host cell into which the recombinant DNA of the present invention is to be introduced may be any of a prokaryote, a yeast, an animal cell, an insect cell, a plant cell, and the like. The host cell is preferably a prokaryote or a yeast strain, is more preferably a prokaryote belonging to the genus Escherichia, the genus Serratia, the genus Bacillus, the genus Brevibacterium, the genus Corynebacterium, the genus Microbacterium, the genus Pseudomonas, or the like, or a yeast strain belonging to the genus Saccharomyces, the genus Schizosaccharomyces, the genus Kluyveromyces, the genus Trichosporon, the genus Siwaniomyces, the genus Pichia, the genus Candida, or the like, and is most preferably a prokaryote such as Escherichia coli BL21 codon plus, Escherichia coli XL1-Blue, and Escherichia coli XL2-Blue (all manufactured by Agilent Technologies, Inc.), Escherichia coli BL21 (DE3) pLysS (manufactured by Merck Millipore Inc.), Escherichia coli DH5α, Escherichia coli HST08 Premium, Escherichia coli HST02, Escherichia coli HST04 dam⁻/dcm⁻, Escherichia coli JM109, Escherichia coli HB101, Escherichia coli CJ236, Escherichia coli BMH71-18 mutS, Escherichia coli MV1184, and Escherichia coli TH2 (all manufactured by Takara Bio Inc.), Escherichia coli W, Escherichia coli JM101, Escherichia coli W3110, Escherichia coli MG1655, Escherichia coli DH1, Escherichia coli MC1000, Escherichia coli W1485, Escherichia coli MP347, Escherichia coli NM522, Escherichia coli ATCC9637, Escherichia coli KY3591 (Deposit No. NITE BP-03062), Serratia ficaria, Serratia fonticola, Serratia liquefaciens, Serratia marcescens, Bacillus subtilis, Bacillus amyloliquefaciens, Brevibacterium immariophilum ATCC 14068, Brevibacterium saccharolyticum ATCC14066, Corynebacterium ammoniagenes, Corynebacterium glutamicum ATCC13032, Corynebacterium glutamicum ATCC14067, Corynebacterium glutamicum ATCC13869, Corynebacterium acetoacidophilumATCC13870, Microbacterium ammoniaphilum ATCC15354], or Pseudomonas sp. D-0110, or a yeast strain such as Saccharomyces cerevisiae, Schizosaccharomyces pombe, Kluyveromyces lactis, Trichosporon pullulans, Schwanniomyces alluvius, Pichia pastoris, or Candida utilis.

The above Escherichia coli KY3591 is deposited on a National Institute of Technology and Evaluation (NITE), Patent Microorganisms Depositary (NPMD) located at 2-5-8 Kazusakamatari, Kisarazu-shi, Chiba, Japan (zip code 292-0818). The date of receipt (date of deposit) is November 18, 2019, and the deposit number is NITE BP-03062.

The host cell is preferably a breeding strain that is artificially endowed or enhanced with an ability to produce GDP-fucose, which is a reaction substrate of α1,2-fucosyltransferase.

Examples of the method for artificially endowing or enhancing the ability to produce GDP-fucose from a saccharide to a microorganism used as a host cell include (a) a method of alleviating or releasing at least one mechanism for controlling a biosynthetic pathway for producing GDP-fucose from a saccharide, (b) a method of enhancing expression of at least one enzyme associated with a biosynthetic pathway for producing GDP-fucose from a saccharide, (c) a method of increasing the number of copies of at least one gene encoding an enzyme associated with a biosynthetic pathway for producing GDP-fucose from a saccharide, and (d) a method of weakening or blocking at least one metabolic pathway branching off from a biosynthetic pathway for producing GDP-fucose from a saccharide to a metabolic product other than a target substance, and the above known methods can be used alone or in combination.

Specific examples of the mechanism for controlling a biosynthetic pathway for producing GDP-fucose from a saccharide include known mechanisms such as a control mechanism based on a transcription control factor (RcsA) associated with control of the biosynthetic pathway.

Specific examples of the enzyme associated with the biosynthetic pathway for producing GDP-fucose from a saccharide include known enzymes such as a mannose-6-phosphate isomerase, a phosphomannomutase, a mannose-1-phosphate guanylyltransferase, a GDP mannose-4,6-dehydratase, and a GDP-L-fucose synthase.

Specific examples of the metabolic pathway branching off from a biosynthetic pathway for producing GDP-fucose from a saccharide to a metabolic product other than a target substance include a known metabolic pathway such as a metabolic pathway from GDP-fucose to comanic acid.

Specific examples of the method for endowing or enhancing the ability to produce GDP-fucose include a known method such as a method using various genetic manipulations (Metabolic Engineering (2017) 41: 23-38).

As the host cell, a breeding strain that is artificially endowed or enhanced with an ability to to supply a receptor carbohydrate, which is a reaction substrate for α1,2-fucosyltransferase can also be used.

Examples of the method for artificially endowing or enhancing the ability to supply a receptor carbohydrate to a microorganism used as a host cell include (a) a method of alleviating or releasing at least one mechanism for controlling a biosynthetic pathway for producing a receptor carbohydrate from a saccharide, (b) a method of enhancing expression of at least one enzyme associated with a biosynthetic pathway for producing a receptor carbohydrate from a saccharide, (c) a method of increasing the number of copies of at least one gene encoding an enzyme associated with a biosynthetic pathway for producing a receptor carbohydrate from a saccharide, (d) a method of alleviating or releasing at least one mechanism for decomposing a receptor carbohydrate, (e) a method of enhancing an expression of at least one enzyme associated with intracellular uptake of a receptor carbohydrate, (f) a method of increasing the number of copies of at least one gene encoding an enzyme associated with intracellular uptake of a receptor carbohydrate, and (g) a method of weakening or blocking at least one metabolic pathway branching off from a receptor carbohydrate to a metabolic product other than a target substance, and the above known methods can be used alone or in combination.

Specific examples of the enzyme associated with a biosynthetic pathway for producing a receptor carbohydrate from a saccharide include known enzymes such as an enzyme having a lactose synthase activity for producing lactose using glucose and UDP-galactose as substrates. Specific examples of the mechanism for decomposing a receptor carbohydrate include known enzymes such as β-galactosidase that catalyzes hydrolysis of lactose to produce glucose and galactose.

Specific examples of the enzyme associated with intracellular uptake of a receptor carbohydrate include known enzymes such as lactose permease associated with intracellular uptake of lactose.

Specific examples of the method for endowing or enhancing the ability to supply a receptor carbohydrate include known methods such as a method of reducing or inactivating an activity of β-galactosidase by a genetic manipulation (Metabolic Engineering (2017) 41: 23-38).

Examples of the method for introducing the recombinant DNA of the above 3 as an autonomously replicatable plasmid in a host cell include the above-described method using calcium ions, protoplast method, and electroporation method, a spheroplast method [Proc. Natl. Acad. Sci., USA, 81, 4889 (1984)], and a lithium acetate method [J. Bacteriol., 153, 163 (1983)].

Examples of the method for incorporating a recombinant DNA into a chromosome of a host cell include a homologous recombination method. Examples of the homologous recombination method include a method using a plasmid for homologous recombination that can be prepared by ligating with a plasmid DNA comprising a drug resistance gene that cannot autonomously replicate in a host cell to be introduced. Examples of the method using homologous recombination frequently used in Escherichia coli include a method of introducing a recombinant DNA using a homologous recombination system of a lambda phage [Proc. Natl. Acad. Sci. USA, 97, 6641-6645 (2000)].

Further, by using a selection method based on the fact that Escherichia coli becomes sucrose-sensitive due to a Bacillus subtilis revansucrase incorporated on the chromosome together with a recombinant DNA, or a selection method based on the fact that Escherichia coli becomes streptomycin-sensitive by incorporating a wild-type rpsL gene into Escherichia coli comprising a mutant rpsL gene for streptomycin resistance [Mol. Microbiol., 55, 137 (2005), Biosci. Biotechnol. Biochem., 71, 2905 (2007)], Escherichia coli with a target region on a chromosomal DNA of the host cell substituted with the recombinant DNA can be obtained.

The fact that the transformant obtained by the above method comprises the DNA of the present invention according to the above 2 can be confirmed by, for example, in the case where the transformant is a transformant obtained by transforming a host cell having an ability to produce GDP-fucose or a receptor carbohydrate from a saccharide, culturing the transformant in a culture medium, and comparing the production amount of fucose-containing carbohydrate accumulated in the culture product with that of a parent strain. It can also be confirmed by preparing an extract containing the protein of the present invention from the culture product, causing the extract, GDP-fucose, and a receptor carbohydrate to exist in an aqueous medium, and comparing the production amount of fucose-containing carbohydrate accumulated in the aqueous medium with that of a parent strain.

Examples of such a transformant of the present invention include the WFL/pAHY2 strain, the WFL/pAHY3 strain, thr WFL/pAHY4 strain, and the WFL/pAHY5 strain to be described later in Examples.

### 5. Method for Producing Fucose-containing Carbohydrate of Present Invention

The method for producing a fucose-containing carbohydrate of the present invention is a method according to the following 5-1 and 5-2.

### 5-1. Method for Producing Fucose-containing Carbohydrate by Fermentation Method

Examples of the method for producing a fucose-containing carbohydrate of the present invention include a method for producing a fucose-containing carbohydrate by a fermentation method including culturing the transformant of the above 4 in a culture medium to produce a fucose-containing carbohydrate in a culture product.

The transformant of the present invention used in the method for producing a fucose-containing carbohydrate by a fermentation method is preferably a transformant having an ability to produce GDP-fucose from a saccharide.

As the transformant of the present invention used in the method for producing a fucose-containing carbohydrate by a fermentation method, a transformant having an ability to produce a receptor carbohydrate may be used.

The method of culturing the transformant in the above 4 can be performed according to a method generally used for culturing a microorganism.

As a culture medium for culturing the transformant, any of a natural culture medium and a synthetic culture medium may be used as long as the culture medium contains a carbon source, a nitrogen source, an inorganic salt, and the like that can be assimilated by the transformant and can efficiently culture the transformant.

Any carbon source may be used as long as it can be assimilated by the transformant, and examples thereof include saccharides such as glucose, fructose, sucrose, molasses containing these, starch, or a starch hydrolysate, organic acids such as acetic acid or propionic acid, or alcohols such as glycerol, ethanol, or propanol.

Examples of the nitrogen source include ammonium salts of inorganic acids or organic acids such as ammonia, ammonium chloride, ammonium sulfate, ammonium acetate, or ammonium phosphate, other nitrogen-containing compounds thereof, peptone, a meat extract, a yeast extract, a corn steep liquor, a casein hydrolysate, a soybean meal, a soybean meal hydrolysate, various fermented bacterial cells, and digestive products thereof.

Examples of the inorganic salt include potassium primary phosphate, potassium secondary phosphate, magnesium phosphate, magnesium sulfate, sodium chloride, ferrous sulfate, manganese sulfate, copper sulfate, and calcium carbonate.

A receptor carbohydrate such as lactose as a precursor of a fucose-containing carbohydrate may be added to the culture medium, or GTP, mannose, or the like as a precursor of GDP-fucose may be added to the culture medium.

In the method for producing a fucose-containing carbohydrate by a fermentation method, when the transformant to be used does not have the ability to produce GDP-fucose, GDP-fucose is added to the culture medium during culture.

In the method for producing a fucose-containing carbohydrate by a fermentation method, when the transformant to be used does not have the ability to produce GDP-fucose, instead of adding GDP-fucose to the culture medium, GDP-fucose may be supplied to the transformant of the present invention by culturing microorganisms having the ability to produce GDP-fucose from a saccharide simultaneously with the transformant of the present invention.

In order to supply or enhance GTP, which is a precursor of GDP-fucose, microorganisms having the ability to produce GTP may be simultaneously cultured. Examples of the microorganism having the ability to produce GTP include known microorganisms such as a microorganism in which expression of an enzyme associated with a biosynthetic pathway of GTP is enhanced by various genetic manipulations (Biotechnol Bioeng (2019) Sep3).

In the method for producing a fucose-containing carbohydrate by a fermentation method, when the transformant to be used does not have the ability to produce a receptor carbohydrate such as lactose, a receptor carbohydrate such as lactose is added to the culture medium during culture.

In the method for producing a fucose-containing carbohydrate by a fermentation method, when the transformant to be used does not have the ability to produce a receptor carbohydrate such as lactose, instead of adding a receptor carbohydrate such as lactose to the culture medium during culture, a receptor carbohydrate such as lactose may be supplied to the transformant of the present invention by culturing microorganisms having the ability to produce a receptor carbohydrate such as lactose from a saccharide simultaneously with the transformant of the present invention.

The culture is generally preferably performed under preferred conditions such as shaking culture or deep aeration stirring culture. The culture temperature is generally 15°C to 40°C, and the culture time is generally 5 hours to 7 days. The pH of the culture solution during culture is generally maintained at 3.0 to 9.0. The pH is adjusted using an inorganic or organic acid, an alkaline solution, urea, calcium carbonate, ammonia, or the like.

If necessary, antibiotics such as ampicillin and tetracycline may be added to the culture medium during the culture. When culturing a microorganism transformed with an expression vector using an inducible promoter as a promoter, an inducer may be added to the culture medium as necessary. For example, isopropyl-β-D-thiogalactopyranoside (IPTG) or the like may be added to the culture medium when culturing a microorganism transformed with an expression vector using a lac promoter, and indole acrylic acid or the like may be added to the culture medium when culturing a microorganism transformed with an expression vector using a trp promoter.

A fucose-containing carbohydrate can be produced by producing and accumulating a fucose-containing carbohydrate in a culture product by the above culture and collecting the fucose-containing carbohydrate from the culture product.

The obtained fucose-containing carbohydrate can be analyzed by a general method using carbohydrate ion chromatography or the like. The collection of the fucose-containing carbohydrate from the culture product or the processed product of the culture product can be performed by a general method using activated carbon, an ion exchange resin, or the like. When the fucose-containing carbohydrate is accumulated in bacterial cells, for example, the fucose-containing carbohydrate can be collected, by using activated carbon, an ion exchange resin, or the like, from a supernatant obtained by crushing the bacterial cells by ultrasonic waves or the like and removing the bacterial cells by centrifugation.

### 5-2. Method for Producing Fucose-containing Carbohydrate using GDP-fucose and Receptor Carbohydrate as Substrates

Examples of the method for producing a fucose-containing carbohydrate of the present invention include a method for producing a fucose-containing carbohydrate using GDP-fucose and a receptor carbohydrate as precursors.

Specifically, a culture product obtained by culturing the transformant of the above 4 or a processed product of the culture product can be used as an enzyme source, the enzyme source, GDP-fucose, and a receptor carbohydrate can be caused to exist in an aqueous medium to produce and accumulate a fucose-containing carbohydrate in the aqueous medium, and the fucose-containing carbohydrate can be collected from the aqueous medium.

The GDP-fucose and the receptor carbohydrate are not limited as long as they can serve as substrates of the protein of the present invention possessed by the transformant of the present invention, and a culture product of a microorganism having an ability to produce GDP-fucose or a receptor carbohydrate, or a processed product of the culture product may be used as it is, or the GDP-fucose and the receptor carbohydrate collected from the culture product or the processed product of the culture product may be used.

Examples of the processed product of the culture product include a concentrate of the culture product, a dried product of the culture product, a bacterial cell obtained by centrifuging the culture product, a dried product of the bacterial cell, a freeze-dried product of the bacterial cell, a surfactant processed product of the bacterial cell, an ultrasonic processed product of the bacterial cell, a mechanical trituration processed product of the bacterial cell, a solvent processed product of the bacterial cell, an enzyme processed product of the bacterial cell, a protein fraction of the bacterial cell, an immobilized product of the bacterial cell, and an enzyme target obtained by extraction from the bacterial cell.

Examples of the aqueous medium include water, phosphate, carbonate, acetate, borate, citrate, buffers such as Tris, alcohols such as methanol and ethanol, esters such as ethyl acetate, ketones such as acetone, and amides such as acetamide. For example, a culture solution of a microorganism used as an enzyme source may be used as an aqueous medium.

The method for analyzing and collecting the fucose-containing carbohydrate produced in the aqueous medium is the same as that in 5-1.

### [Analysis Example]

In Examples, analysis and quantification of 2'-fucosyllactose were performed by the following procedure. A culture solution containing microorganisms after culture was centrifuged, and the supernatant was collected. 2'-Fucosyllactose contained in the supernatant was analyzed by a carbohydrate analyzer ICS-5000 (manufactured by Thermo Fisher Scientific K.K.).

### [Analysis Conditions]

Column: CarboPAC PA1
Column temperature: 25°C
Mobile phase: (mobile phase A) water
(mobile phase B) 500 mmol/L sodium hydroxide
(mobile phase C) 300 mmol/L sodium acetate
Mobile phase A, mobile phase B, and mobile phase C mixing ratio:
(0 minutes to 10 minutes) gradient from 80:20:0 to 70:20:10
(10 minutes to 18 minutes) 70:20:10
(18 minutes to 25 minutes) 80:20:0
Flow rate: 0.8 mL/min
Detector: pulsed amperometric detector

Example

Examples of the present invention are shown below, but the invention is not limited to these Examples.

### [Example 1] Construction of microorganism used for production of 2'-fucosyllactose

### (1) Acquisition of DNA fragment to be used as marker for gene deletion

PCR was performed using, as a primer set, DNAs consisting of the nucleotide sequences shown in "Primer set" in Table 1 and using, as a template, a DNA described in "Template" in Table 1 to obtain each amplified DNA fragment.

**[Table 1]**

| Primer set (SEQ ID NO:) | Template | Amplified DNA fragment |
|---|---|---|
| 15 and 16 | pHSG396 (manufactured by Takara Bio Inc.) | cat |
| 17 and 18 | Genomic DNA of Bacillus subtilis 168 strain | sacB |

A genomic DNA of the Bacillus subtilis 168 strain was prepared by a standard method. The cat of the amplified DNA fragment comprises about 200 bp upstream to about 50 bp downstream of the cat gene on pHSG 396. The sacB of the amplified DNA fragment comprises about 300 bp upstream to about 100 bp downstream of the sacB gene on the genomic DNA of the Bacillus subtilis 168 strain.

Next, PCR was performed using, as a template, the cat and sacB of the amplified DNA fragment and using, as a primer set, DNAs consisting of the nucleotide sequences represented by SEQ ID NOs: 15 and 18 to obtain a DNA fragment comprising the cat gene and the sacB gene (hereinafter, referred to as cat-sacB).

### (2) Construction of Escherichia coli with loss of β-galactosidase activity, lactose permease activity, and colanic acid producing activity

Escherichia coli deficient in DNA encoding β-galactosidase (hereinafter, referred to as lacZ gene), DNA encoding lactose permease (hereinafter, referred to as lacY gene), and DNA encoding a colanic acid production-related protein (hereinafter, referred to as wcaJ, wzxC, wcaK, wcaL, or wcaM gene) was constructed by the following method. Note that the lacZ and lacY (hereinafter, referred to as lacZY), and wcaJ, wzxC, wcaK, wcaL, and wcaM (hereinafter, referred to as wcaJ-wzxC-wcaKLM) each form an operon on the Escherichia coli genome.

PCR was performed using, as a template, a genomic DNA of the Escherichia coli KY3591 strain (Deposit No. NITE BP-03062) prepared by an ordinary method and using, as a primer set, DNAs consisting of the nucleotide sequences shown in "Primer set" in Table 2 to amplify each DNA fragment.

**[Table 2]**

| Primer set (SEQ ID NO:) | Amplified DNA fragment | Remark |
|---|---|---|
| 19 and 20 | lacZ upstream 1 | Sequences at 5' ends of nucleotide sequences represented by SEQ ID NOs: 15 and 19 are complementary |
| 21 and 22 | lacY downstream 1 | Sequences at 5' ends of nucleotide sequences represented by SEQ ID NOs: 18 and 21 are complementary |
| 20 and 23 | lacZ upstream 2 | Sequences at 5' ends of nucleotide sequences represented by SEQ ID NOs: 23 and 24 are complementary |
| 22 and 24 | lacY downstream 2 | |

The lacZ upstream 1 and lacZ upstream 2 comprise about 900 bp upstream from an initiation codon of the lacZ gene. The lacY downstream 1 and lacY downstream 2 comprise about 800 bp downstream from a stop codon of the lacY gene.

PCR was performed using, as a template, a mixture of the lacZ upstream 1, the lacY downstream 1, and the cat-sacB fragment at an equimolar ratio and using, as a primer set, DNAs consisting of the nucleotide sequences represented by SEQ ID NOs: 20 and 22 to obtain a DNA fragment consisting of a sequence with the cat-sacB fragment inserted in a region around the lacZ and lacY genes (hereinafter, referred to as lacZY::cat-sacB).

PCR was performed using, as a template, a mixture of the lacZ upstream 2 and the lacY downstream 2 at an equimolar ratio and using, as a primer set, DNAs consisting of the nucleotide sequences represented by SEQ ID NOs: 20 and 22 to obtain a DNA fragment consisting of a sequence with the lacZ upstream and the lacY downstream directly linked to each other without lacZ and lacY (hereinafter, referred to as ΔlacZY).

The lacZY::cat-sacB fragment was introduced into Escherichia coli carrying a plasmid pKD46 comprising a gene encoding λ recombinase [Datsenko, K. A., Warner, B. L. L., Proc. Natl. Acad.Sci, USA, Vol. 97, 6640-6645, (2000)] by an electroporation method to obtain a transformant (a transformant with the lacZ and lacY genes substituted with lacZY::cat-sacB) exhibiting chloramphenicol resistance and sucrose sensitivity.

The ΔlacZY fragment was introduced into the transformant by the electroporation method to obtain a transformant (a transformant with lacZY::cat-sacB substituted with ΔlacZY) exhibiting chloramphenicol sensitivity and sucrose resistance. Among them, a transformant (a transformant without pKD46) exhibiting ampicillin sensitivity was further obtained. The transformant was named the ΔlacZY.

Similarly, PCR was performed using, as a template, a genomic DNA of the Escherichia coli KY3591 strain and using, as a primer set, DNAs consisting of the nucleotide sequence shown in "Primer set" in Table 3 to obtain each amplified DNA fragment.

**[Table 3]**

| Primer set (SEQ ID NO:) | Amplified DNA fragment | Remark |
|---|---|---|
| 25 and 26 | wcaJ upstream 1 | Sequences at 5' ends of nucleotide sequences represented by SEQ ID NOs: 15 and 25 are complementary |
| 27 and 28 | wcaM downstream 1 | Sequences at 5' ends of nucleotide sequences represented by SEQ ID NOs: 18 and 27 are complementary |
| 26 and 29 | wcaJ upstream 2 | Sequences at 5' ends of nucleotide sequences represented by SEQ ID NOs: 29 and 30 are complementary |
| 28 and 30 | wcaM downstream 2 | |

The wcaJ upstream 1 and the wcaJ upstream 2 comprise about 900 bp upstream from an initiation codon of the wcaJ gene. The wcaM downstream 1 and the wcaM downstream 2 comprise about 800 bp downstream from a stop codon of the wcaM gene.

PCR was performed using, as a template, a mixture of the wcaJ upstream 1, the wcaM downstream 1, and the cat-sacB fragment at an equimolar ratio and using, as a primer set, DNAs consisting of the nucleotide sequences represented by SEQ ID NOs: 26 and 28 to obtain a DNA fragment consisting of a sequence with the cat-sacB fragment inserted in a region around a wcaJ-wzxC-wcaKLM operand (hereinafter, referred to as wcaJ-wzxC-wcaKLM::cat-sacB).

PCR was performed using, as a template, a mixture of the wcaJ upstream 2 and the wcaM downstream 2 at an equimolar ratio and using, as a primer set, DNAs consisting of the nucleotide sequences represented by SEQ ID NOs: 26 and 28 to obtain a DNA fragment consisting of a sequence with the wcaJ upstream and the wcaM downstream directly linked to each other without wcaJ-wzxC-wcaKLM (hereinafter, referred to as ΔwcaJ-wzxC-wcaKLM).

The wcaJ-wzxC-wcaKLM::cat-sacB fragment was introduced into the ΔlacZY strain constructed as described above by the electroporation method to obtain a transformant (a transformant with wcaJ-wzxC-wcaKLM substituted with wcaJ-wzxC-wcaKLM::cat-sacB) exhibiting chloramphenicol resistant and sucrose sensitivity.

The ΔwcaJM fragment was introduced into the transformant by the electroporation method to obtain a transformant (a transformant with wcaJ-wzxC-wcaKLM::cat-sacB substituted with ΔwcaJ-wzxC-wcaKLM) exhibiting chloramphenicol sensitivity and sucrose resistance. Further, a transformant (a transformant without pKD46) exhibiting ampicillin sensitivity was obtained. The transformant was named the WFL strain.

### (3) Preparation of Helicobacter mustelae-derived α1,2-fucosyltransferase (HMFT) expression vector

PCR was performed using, as a primer set, DNAs consisting of the nucleotide sequences shown in "Primer set" in Table 4 and using, as a template, a DNA described in "Template" in Table 4 to obtain each amplified DNA fragment.

**[Table 4]**

| Primer set (SEQ ID NO:) | Template | Amplified DNA fragment |
|---|---|---|
| 3 1 and 32 | Genomic DNA of Escherichia coli W3110 strain | rcsA |
| 33 and 34 | pHMFT (Japanese Patent No. 4048173) | HMFT |
| 35 and 36 | Genomic DNA of Escherichia coli W3110 strain | lacY |

A genomic DNA of the Escherichia coli W3110 strain was prepared by an ordinary method. The nucleotide sequences represented by SEQ ID NOs: 32 and 33 and SEQ ID NOs: 34 and 35 comprise complementary sequences at each 5' end.

PCR was performed using, as a template, a mixture of the rcsA, HMFT, and lacY fragments at an equimolar ratio and using, as a primer set, DNAs consisting of the nucleotide sequences represented by SEQ ID NOs: 37 and 38 to obtain a DNA fragment with the three fragments linked (hereinafter, referred to as rcsA-HMFT-lacY).

PCR was performed using, as a primer set, DNAs consisting of the nucleotide sequences represented by SEQ ID NOs: 39 and 40 and using, as a template, a plasmid pPE 167 (Appl. Environ. Microbiol. 2007, 73: 6378-6385) to obtain a vector fragment of about 4.4 kb.

In this case, the nucleotide sequences represented by SEQ ID NOs: 37 and 40 and SEQ ID NOs: 38 and 39 comprise complementary sequences at each 5' end.

The rcsA-HMFT-lacY fragment and the vector fragment obtained above were ligated using an In-Fusion HD Cloning Kit (manufactured by Takara Bio Inc.) to obtain an expression plasmid pAHY1.

### (4) Construction of microorganism expressing mutant HMFT

Using, as a template, the plasmid pAHY1 obtained in the above (3), using a Prime STAR Mutagenesis Basal Kit (manufactured by Takara Bio Inc.), and using, as a primer set, DNAs consisting of the nucleotide sequence shown in "Primer set" in Table 5, a plasmid pAHY2 was prepared by introducing all the five amino acid mutations into the amino acid sequence of HMFT.

Similarly, a plasmid pAHY3 was prepared by introducing a mutation to an amino acid residue at a position 9 in the amino acid sequence of HMFT.

Similarly, a plasmid pAHY4 was prepared by introducing a mutation to an amino acid residue at a position 238 in the amino acid sequence of HMFT.

Similarly, a plasmid pAHY5 was prepared by introducing mutations to amino acid residues at positions 9 and 238 in the amino acid sequence of HMFT.

**[Table 5]**

| Amino acid position | Amino acid | Primer set (SEQ ID NO:) | Amino acid mutation |
|---|---|---|---|
| Position 9 | L-His | 41 and 42 | H9R |
| Position 68 | L-His | 43 and 44 | H68R |
| Position 75 | L-Leu | 45 and 46 | L75P |
| Position 86 | Gly | 47 and 48 | G86S |
| Position 238 | L-Thr | 49 and 50 | T238A |

Using the plasmid pAHYl obtained in (3) and the above pAHY2, pAHY3, pAHY4, and pAHY5, the WFL strain constructed in the above (2) was transformed, and the WFL/pAHY1 strain, the WFL/pAHY2 strain, the WFL/pAHY3 strain, the WFL/pAHY4 strain, and the WFL/pAHY5 strain were obtained.

### [Example 2] Production of 2'-fucosyllactose by fermentation method using microorganism expressing mutant HMFT

The WFL/pAHY1 strain, the WFL/pAHY2 strain, the WFL/pAHY3 strain, the WFL/pAHY4 strain, and the WFL/pAHY5 strain obtained in Example 1 were incubated on LB plates at 30°C for 24 hours, inoculated into large-sized test tubes each containing 5 mL of an LB culture medium containing 100 mg/L of kanamycin, and shaking-cultured at 30°C for 16 hours. Thereafter, 0.1 mL of each obtained culture solution was inoculated into a large-sized test tube containing 5 mL of a production culture medium [glucose 30 g/L, lactose monohydrate 5 g/L, magnesium sulfate heptahydrate 2 g/L, dipotassium hydrogen phosphate 16 g/L, potassium dihydrogen phosphate 14 g/L, ammonium sulfate 2 g/L, citric acid monohydrate 1 g/L, casamino acid 5 g/L, thiamine hydrochloride 10 mg/L, ferrous sulfate heptahydrate 50 mg/L, manganese sulfate pentahydrate 10 mg/L (except for glucose, lactose monohydrate, and magnesium sulfate heptahydrate, which were dissolved in sodium hydroxide solution at pH 7.2, and then autoclaved) (aqueous solutions containing glucose, lactose monohydrate, and magnesium sulfate heptahydrate were separately prepared, autoclaved, cooled, and then mixed)] containing 100 mg/L of kanamycin, and shaking-cultured at 30°C for 30 hours.

After completion of the culture, the culture solution was appropriately diluted and centrifuged, and 2'-fucosyllactose contained in the supernatant was analyzed by HPLC. The results are shown in Table 6.

**[Table 6]**

| Strain name | Amino acid mutation | 2'-fucosyllactose (g/L) |
|---|---|---|
| WFL/pAHY1 | - | 2.21 |
| WFL/pAHY2 | H9R, H68R, L75P, G86S, T238A | 2.46 |
| WFL/pAHY3 | H9R | 2.96 |
| WFL/pAHY4 | T238A | 2.69 |
| WFL/pAHY5 | H9R, T238A | 3.38 |

As shown in Table 6, the WFL/pAHY2 strain, the WFL/pAHY3 strain, the WFL/pAHY4 strain, and the WFL/pAHY5 strain showed higher 2'-fucosyllactose productivity as compared to the WFL/pAHY1 strain.

From the above, it is found that the 2'-fucosyllactose productivity is improved by using a microorganism containing a mutant HMFT compared to a microorganism containing a wild-type HMFT.

### Industrial Applicability

According to the present invention, a modified protein having an α1,2-fucosyltransferase activity and a method for producing a fucose-containing carbohydrate using the protein or a microorganism having an ability to produce the protein are provided.

Although the present invention has been described in detail with reference to specific embodiments, it will be apparent to those skilled in the art that various changes and modifications can be made without departing from the spirit and scope of the invention. The present application is based on a Japanese Patent Application No. 2021-025170 filed on February 19, 2021, the entire contents of which are incorporated herein by reference. All references cited herein are incorporated in their entirety.

## Claims

1. A protein having an improved α1,2-fucosyltransferase activity compared to an original protein according to any one of [1] to [3] below and consisting of an amino acid sequence in which one or more amino acid residues selected from the group consisting of amino acid residues corresponding to the amino acid residues at positions 9, 68, 75, 86, and 238 in the amino acid sequence represented by SEQ ID NO: 2 are substituted with another amino acid residue in an amino acid sequence of the protein according to any one of [1] to [3] below,
[1] a protein consisting of the amino acid sequence represented by SEQ ID NO: 2, 4, 6, 8, or 10,
[2] a mutant protein having an α1,2-fucosyltransferase activity and consisting of an amino acid sequence in which 1 to 20 amino acids are deleted, substituted, inserted, and/or added in the amino acid sequence represented by SEQ ID NO: 2, 4, 6, 8, or 10, and
[3] a homologous protein having an α1,2-fucosyltransferase activity and consisting of an amino acid sequence having an identity of 80% or more with the amino acid sequence represented by SEQ ID NO: 2, 4, 6, 8, or 10.

2. The protein according to claim 1, wherein the protein consists of an amino acid sequence in which the amino acid residues corresponding to the amino acid residues at positions 9, 68, 75, 86, and 238 in the amino acid sequence represented by SEQ ID NO: 2 are substituted with another amino acid residue in the amino acid sequence of the protein according to any one of [1] to [3].

3. The protein according to claim 1 or 2, wherein the protein consists of an amino acid sequence with at least one substitution selected from the following [1a] to [1e] in the amino acid sequence of the protein according to any one of [1] to [3],
[1a] the amino acid residue corresponding to the amino acid residue at position 9 in the amino acid sequence represented by SEQ ID NO: 2 being substituted with L-lysine or L-arginine,
[1b] the amino acid residue corresponding to the amino acid residue at position 68 in the amino acid sequence represented by SEQ ID NO: 2 being substituted with L-lysine or L-arginine,
[1c] the amino acid residue corresponding to the amino acid residue at position 75 in the amino acid sequence represented by SEQ ID NO: 2 being substituted with L-proline,
[1d] the amino acid residue corresponding to the amino acid residue at position 86 in the amino acid sequence represented by SEQ ID NO: 2 being substituted with L-threonine or L-serine, and
[1e] the amino acid residue corresponding to the amino acid residue at position 238 in the amino acid sequence represented by SEQ ID NO: 2 being substituted with L-leucine, L-isoleucine, or L-alanine.

4. The protein according to any one of claims 1 to 3, wherein the protein consists of an amino acid sequence with at least one substitution selected from the following [2a] to [2e] in the amino acid sequence of the protein according to any one of [1] to [3],
[2a] the amino acid residue corresponding to the amino acid residue at position 9 in the amino acid sequence represented by SEQ ID NO: 2 being substituted with L-arginine,
[2b] the amino acid residue corresponding to the amino acid residue at position 68 in the amino acid sequence represented by SEQ ID NO: 2 being substituted with L-arginine,
[2c] the amino acid residue corresponding to the amino acid residue at position 75 in the amino acid sequence represented by SEQ ID NO: 2 being substituted with L-proline,
[2d] the amino acid residue corresponding to the amino acid residue at position 86 in the amino acid sequence represented by SEQ ID NO: 2 being substituted with L-serine, and
[2e] the amino acid residue corresponding to the amino acid residue at position 238 in the amino acid sequence represented by SEQ ID NO: 2 being substituted with L-alanine.

5. The protein according to any one of claims 1 to 4, wherein the protein consists of an amino acid sequence in which the amino acid residue corresponding to the amino acid residue at position 9 in the amino acid sequence represented by SEQ ID NO: 2 is substituted with L-arginine in the amino acid sequence of the protein according to any one of [1] to [3].

6. The protein according to any one of claims 1 to 4, wherein the protein consists of an amino acid sequence in which the amino acid residue corresponding to the amino acid residue at position 238 in the amino acid sequence represented by SEQ ID NO: 2 is substituted with L-alanine in the amino acid sequence of the protein according to any one of [1] to [3].

7. The protein according to any one of claims 1 to 4, wherein the protein consists of an amino acid sequence in which the amino acid residue corresponding to the amino acid residue at position 9 in the amino acid sequence represented by SEQ ID NO: 2 is substituted with L-arginine and the amino acid residue corresponding to the amino acid residue at position 238 in the amino acid sequence represented by SEQ ID NO: 2 is substituted with L-alanine in the amino acid sequence of the protein according to any one of [1] to [3].

8. A DNA encoding the protein according to any one of claims 1 to 7.

9. A recombinant DNA comprising the DNA according to claim 8,

10. A transformant obtained by transforming a host cell with the recombinant DNA according to claim 9,

11. A method for producing a fucose-containing carbohydrate, comprising: culturing a microorganism having an ability to produce the protein according to any one of claims 1 to 7 in a culture medium to produce a fucose-containing carbohydrate in a culture product.

12. A method for producing a fucose-containing carbohydrate, comprising: culturing a microorganism having an ability to produce the protein according to any one of claims 1 to 7 in a culture medium, using an obtained culture product or a processed product of the culture product as an enzyme source, and causing the enzyme source, GDP-fucose, and a receptor carbohydrate to exist in an aqueous medium to produce a fucose-containing carbohydrate in the aqueous medium.

13. The method for producing a fucose-containing carbohydrate according to claim 12, wherein the receptor carbohydrate is lactose.

14. The production method according to any one of claims 11 to 13, wherein the fucose-containing carbohydrate is 2'-fucosyllactose.
